# EUROPEAN PATENT APPLICATION

(11) **EP 1 125 545 A2**
(43) Date of publication of application: **22.08.2001**
(21) Application number: 01830093.9
(22) Date of filing: 13.02.2001
(51) Int. Cl.: A61B 5/00

(54) **Lifesaving collar-armlet with radio signalling**

(30) Priority: 18.02.2000 IT LE000005
(71) Applicant: Aguglia, Jorge Miguel, 73100 Lecce (IT)
(72) Inventor: Aguglia, Jorge Miguel, 73100 Lecce (IT)

(57) **Abstract**

A system of biological sensors, equipped with a radio transmitter, which is placed on an armlet, collar or directly on the patient and which analyses some physiological parameters of the same patient.
The analysis of the correlation of the variations of such parameters allows the recognition of:
- Shock phenomenon in its initial situation
- Physiological alterations
and consequently activates all the mechanisms necessary for the safety of the patient, or of what it controls, by a codified alarm, radio transmitted or locally generated.

## Description

### -Progress report

At present there is no such a thing on the market allowing the immediate warning about someone's serious health condition (not being under observation), as a consequence of either an accident or involution of health conditions themselves, with immediate transmission at a distance or local alarm.
At present there are several devices devoted to the safeguard of human safety both in working and home environment but all of them extremely dedicated to specific uses, such as:
- Circuit breaker, safety switch
- Monitoring devices (in hospital environment)
- Warning via telephone line upon instructions from the patient (first aid)
- Safety barriers on working islands (preventive)
- Accident prevention equipment (protective)

### - Description of the invention and tables

The system is made up of a series of sensors able to sense the parameters of interest (the choice of such sensors is influenced also by the offer existing on the market and in the future thanks to the developments of technology we will definitely have more evolved sensors).
It has an autonomous supply system and a transmission and reception system via radio.
The whole is run by a customisable processing unit, able to sense the quantity read by the sensors and then evaluate the necessity to make the alarm transmission.
The transmission is made in a codified way so that it is possible to identify univocally the subject from where the alarm comes.
In those radio connected systems, the correct operation of the apparatus is assured by the periodical transmission of a control signal, containing the status of the parameters sensed in that moment.

### Block diagram of the 'SENSOR' (see table 1 - picture 1)

### Standard structure of the 'SENSOR' (see table 1 - picture 2)

The system can be completed by an external receiver which locating the alarm source (by decoding) carries out the expected actions, such as:
- Machine blockade
- Aid alarm
- Telephone calls
- Etc...

The receiving system can be equipped with a monitor either autonomous or interfaced with a processor in order to keep under control all the parameters of all patients under control.

Block diagram of the receiver. (see table 2 - picture 3)

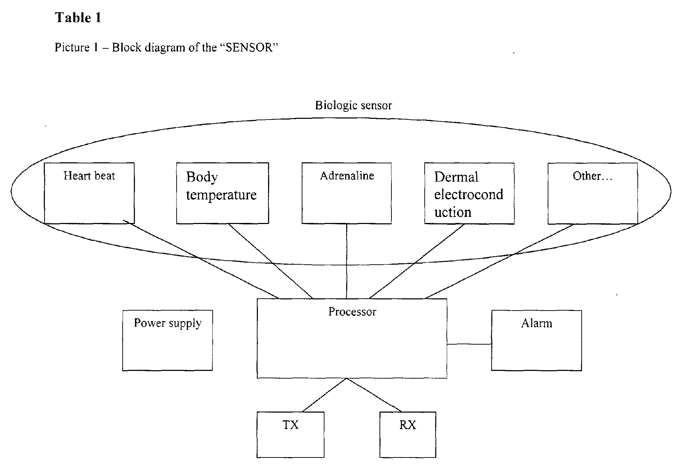

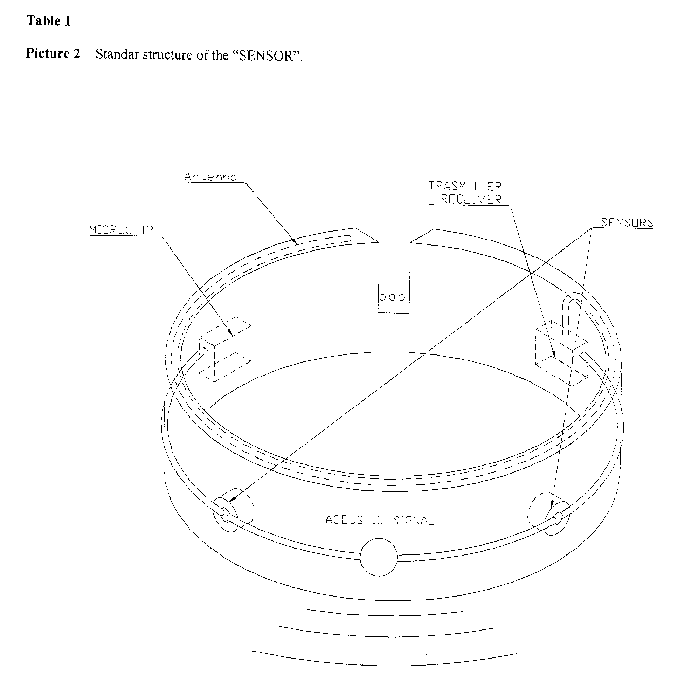

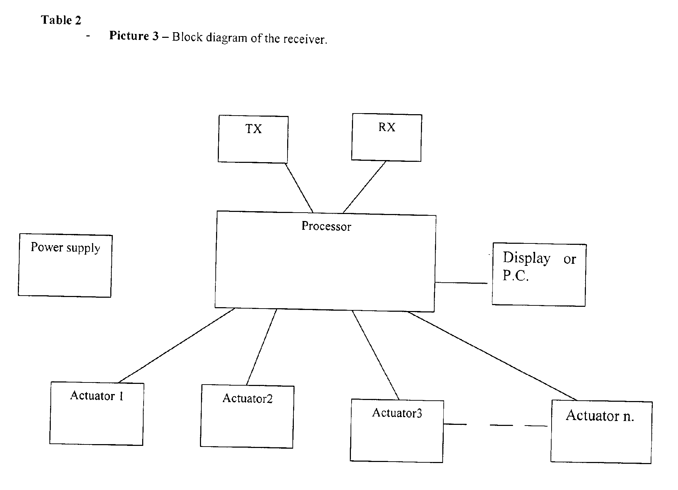

## Claims

1. Autonomous and automatic revelation, transmission and carrying into effect system.

2. System applicable on a support that can be either removable or implanted subcutaneously.

3. In safety at work it is applicable to sense the arousing of accidents and at a preventive level to report a physical condition unfit for the task being carried out.

4. In the automotive sector it is applicable for sensing:
the arising of the 'stroke of sleep' and/or of any situation warning a road accident

5. At home it is applicable for keeping under observation:
- children, elderly, disabled people, patients at risk

6. In the transport sector (buses, trains, aircraft, ships, etc..) in order to act at the right moment in case of sudden illness of the driver

7. It is applicable when advisable to check the emotional conditions of those in charge of the safety of several people (buses, trains, aircraft, ships, etc...) and it acts by the automatic switching on of safety procedures.

8. In nautical, mountain and sporting environment, to sense the situations of man 'overboard' or death by drowning, avalanche, fall, illness, etc.

9. In hospital environment in order to simplify the monitoring of patients, avoiding the cable connection and the patient's call.

10. The versions equipped with an alarm acoustic signal allow the patient to intervene with suitable therapy.

11. In medical environment it is applicable when administering medicines in predetermined doses in relation to the state of need of the patient, in order to intervene actively in situations of the following crisis, such as: epileptic, diabetes, cardiac, hypertensive, etc....

12. In the military sector to monitor the psychophysical condition of people in charge of critical operative functions.

13. the system can be equipped with a self-programming software able to adapt itself to the typical parameters of the subject.

14. The system can be equipped with a communication interface with a cellular telephone in order to self activate telephone calls to previously set emergency numbers or with an interface with the telephone network for the introduction in the network of a codified alarm allowing a fast location of the area of origin.

15. The system can be equipped with a forced activation button and can be used as a beeper using the signal issued by the transmitter.

16. The system can be equipped with a possible external receiver which, locating the alarm source, actuates the scheduled actions, such as: machine stoppage, aid alarm, telephone call, activation of the on board machine devices for the signalling of the danger condition, flashers, bells, etc.
